# EUROPEAN PATENT APPLICATION

(11) **EP 1 611 842 A1**
(43) Date of publication of application: **04.01.2006**
(21) Application number: 05254032.5
(22) Date of filing: 28.06.2005
(51) Int. Cl.: A61B 5/00, A61B 5/15, B29C 65/00, G01N 33/48, G01N 33/52, G01N 33/531

(54) **Apparatus for the manufacture of medical devices**

(30) Priority: 29.06.2004 US 881416
(71) Applicant: LifeScan, Inc., Milpitas, CA 95035 (US)
(72) Inventor: Lang, David K., Inverness, Inverness-shire IV2 4AP (GB); Allen, John, Mendota Heights, Minnesota 55118 (US); Johnson, John, Hendersonville, Tennessee 37075 (US)
(74) Representative: Mercer, Christopher Paul

(57) **Abstract**

The determination of analyte concentration in physiological samples is of ever increasing importance to today's society. Such assays find use in a variety of applications, including clinical laboratory testing, home testing, etc., where the results of such testing play a prominent role in the diagnosis and management of a variety of disease conditions. An apparatus is described herein which may be used for the manufacture of medical devices which include an integrated lancet (220) and sensor (204).

## Description

### BACKGROUND OF THE INVENTION

The present invention relates, in general, to medical devices containing an integrated lancet and sensor and, more particularly, to an assembly apparatus for use in manufacturing such medical devices.

The determination of analyte concentration in physiological samples is of ever increasing importance to today's society. Such assays find use in a variety of applications, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, drugs for monitoring levels of therapeutic agents, and identifying illegal levels of drugs, and the like. In response to this growing importance of analyte concentration determination, a variety of analyte concentration determination protocols and devices for both clinical and home testing have been developed.

In determining the concentration of an analyte in a physiological sample, a physiological sample must first be obtained. Obtaining and testing the sample often involves cumbersome and complicated procedures. Unfortunately, successful manipulation and handling of test elements, such as test strips, lancing members, meters and the like is, to a great extent, dependent on the visual acuity and manual dexterity of the user, which in the case of people with diabetes is subject to deterioration over the course of the disease state. In extreme cases people that have significant loss of sight and sensation, testing procedures can become significantly difficult and require additional assistance from ancillary devices or personnel.

A typical procedure for making a glucose measurement with the use of a test strip involves the following actions or steps (but not necessarily in the order given): (1) removing supplies from a carrying case, (2) removing a lancing device loading cap or door, (3) removing and disposing of a used lancet from the lancing device, (4) inserting the lancet in the lancing device, (5) twisting off a protective cap from the lancet, (6) replacing the lancing device cap, (7) cocking the lancing device, (8) opening a test strip vial/container, (9) removing a strip from the container and inserting or interfacing it with a meter, (10) holding a lancing device to the skin, (11) firing the lancing device, (12) removing the lancing device from the skin, (13) extracting a sample, (14) applying sample to the test strip and obtaining results of the measurement; (15) disposing of the test strip, (16) cleaning the test site, and (17) returning supplies to the carrying case. Of course, certain glucose measurement systems and protocols may involve fewer or more steps.

One manner of reducing the number of actions is by the use of integrated medical devices that combine multiple functions in order to minimize the handling of sensor and/or lancing components that may lead to contamination of the components and/or injury to the user. An example of such an integrated medical device that includes a test strip and lancet is described in International Application No. PCT/GB01/05634 (published as WO 02/49507 on June 27, 2002) and U.S. Patent Publication No. 2002-0168290, both of which are fully incorporated herein by reference.

Technological advancements have been made in test strip fabrication in which both sensor and lancing functions and the structures to provide such functions are provided on a single fully integrated medical device, as described in the aforementioned U.S. Patent Publication No. 2002-0168290. Integrated medical devices are typically in the form of strips. Web-based methods can be used to make such fully integrated medical devices. In these methods, the integrated medical devices are singulated after fabrication prior to being collectively packaged in a cartridge, magazine, cassette or the like. Examples of web-based methods for making such medical devices are disclosed in U.S. Patent Publication No. 2003-0211619 and European Patent Application EP 1360932 A1, both of which are fully incorporated herein by reference. These web-based methods, however, require expensive equipment that requires substantial manufacturing floor space. In web-based methods, the alignment of the sensor and lance can also change during the manufacturing process.

Still needed in the field, therefore, is an inexpensive and simple method of fabricating an integrated medical device containing a lancet and a test strip. This method should also produce integrated medical devices in which the sensor and lance are precisely aligned.

### SUMMARY OF THE INVENTION

In one embodiment of the present invention, an integrated medical device assembly apparatus includes: a body with a proximal end, a distal end, a detachable clamping bar and a pusher plate. In this embodiment of the invention the proximal end of the assembly apparatus includes a plurality of recesses for receiving and removably retaining a plurality of test strips. In an integrated medical device assembly according to the present invention the pusher plate may include a plurality of spring-loaded protrusions for contacting the plurality of test strips retained within the recesses. In an integrated medical device assembly apparatus according to the present invention, the pusher plate may include a resiliently deformable band adapted to force the plurality of test strips retained into the recesses. In an integrated medical device assembly apparatus according to the present invention, the clamping bar includes at least one pin for attaching to the body.

In one embodiment of the present invention an integrated medical device for use in detecting the presence of analytes in blood includes a test strip manufactured using a web process: a first heat activated bonding layer positioned over the first test strip substrate and a tissue penetration member bonded to the test strip by heating the bonding layer. In this embodiment of the present invention, the tissue penetrating member is affixed to the test strip by positioning the test strip and bonding layer in a recessed cavity, placing the tissue penetration member over the bonding layer, clamping the tissue penetration member in place using a clamping bar, forcing the test strip into the recess using a pusher plate and heating the test strip, bonding layer and penetration member to a predetermined temperature. In an integrated medical device according to one embodiment of the present invention the predetermined temperature is between 95°C and 150°C. In an integrated medical device according to one embodiment of the present invention the tissue penetration member includes a lancet. In an integrated medical device according to one embodiment of the present invention, a notch in the distal end of the heat activated bonding layer, a first side of the test strip and a first side of the tissue penetration member form a chamber positioned to receive fluid from the lancet.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings (wherein like numerals represent like elements), of which:

Figure 1 is a partially exploded perspective view of an integrated medical device assembly apparatus according to an embodiment of the present invention;

Figures 2A and 2B are perspective and side views, respectively, of a medical device that can be used with exemplary embodiments of the assembly apparatus according to the present invention; and

Figures 3A and 3B are cross-sectional side views of a portion of the medical device assembly apparatus of Figure 1B along A-A' in the direction of the arrows, representing exemplary embodiments of assembly apparatus recesses.

Figures 4A and 4B are perspective and exploded perspective views, respectively, of an integrated medical device assembly apparatus according to an exemplary embodiment of the present invention;

Figure 5 is a flow chart illustrating a sequence of steps in a process for manufacturing an integrated medical device using the assembly apparatuses according to exemplary embodiments of the present invention;

Figures 6A-6H are schematic, perspective views depicting stages of a process for manufacturing medical devices according to the present invention; and

Figures 7A-7I are schematic, perspective views depicting stages of a process for manufacturing medical devices according to the present invention.

### DETAILED DESCRIPTION OF THE INVENTION

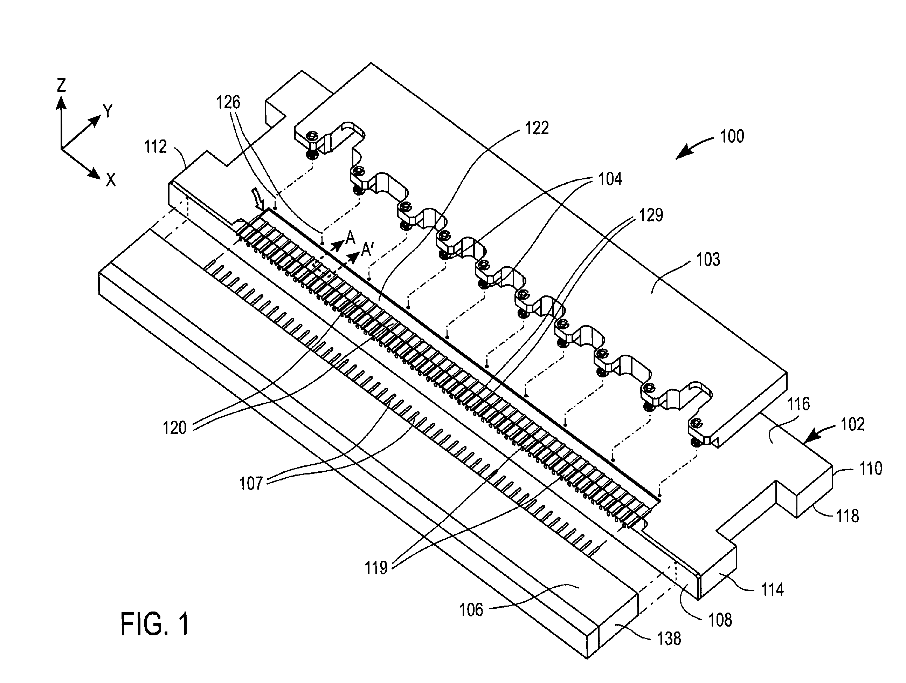

Figure 1 is an exploded perspective view of a medical device assembly apparatus 100 according to an exemplary embodiment of the present invention. Assembly apparatus 100 includes a body 102, a detachable clamping bar 103 with a plurality of locating pins 104, and a detachable test strip pusher plate 106 with a plurality of spring-loaded protrusions 107. Assembly apparatus 100 is generally rectangular in shape and can be formed of metal or any material that can withstand a temperature ranging from about 95°C to 150°C.

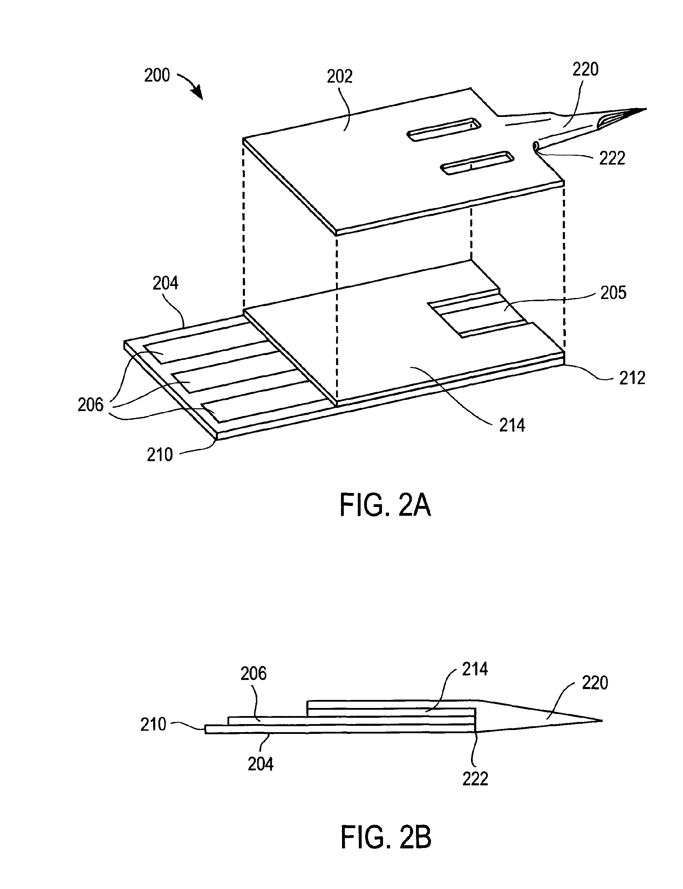

Figures 2A and 2B are perspective and side views, respectively, of an exemplary integrated medical device 200 that can be manufactured using assembly apparatus 100 according to one aspect of the present invention. Integrated medical device 200 includes a test strip 204 and a dermal tissue penetration member 202. Test strip 204 has a reaction area 205 and electrical contacts 206 that terminate on a proximal end 210 of integrated medical device 200. Electrical contacts 206 are made of any suitable conductive material, such as gold, silver, platinum or carbon. Dermal tissue penetration member 202 includes a lancet 220 adapted to pierce a user's skin and draw blood into reaction area 205. Dermal tissue penetration member 202 is adhered to test strip 204 by an adhesive layer 214. This adhesive layer 214 can be heat seal or pressure sensitive adhesive. Lancet 220 includes a lancet base 222 that terminates at the distal end 212 of the assembled test strip. Further descriptions of integrated medical devices that can be manufactured using assembly apparatus 100 according to the present invention are in the aforementioned International Publication No. WO 02/49507 and U.S. Patent Publication No. 2002-0168290. In addition, dermal tissue penetration member 202 can be fabricated, for example, by a progressive die-stamping technique, as disclosed in the aforementioned International Publication No. WO 02/49507 and U.S. Patent Publication No. 2002-0168290.

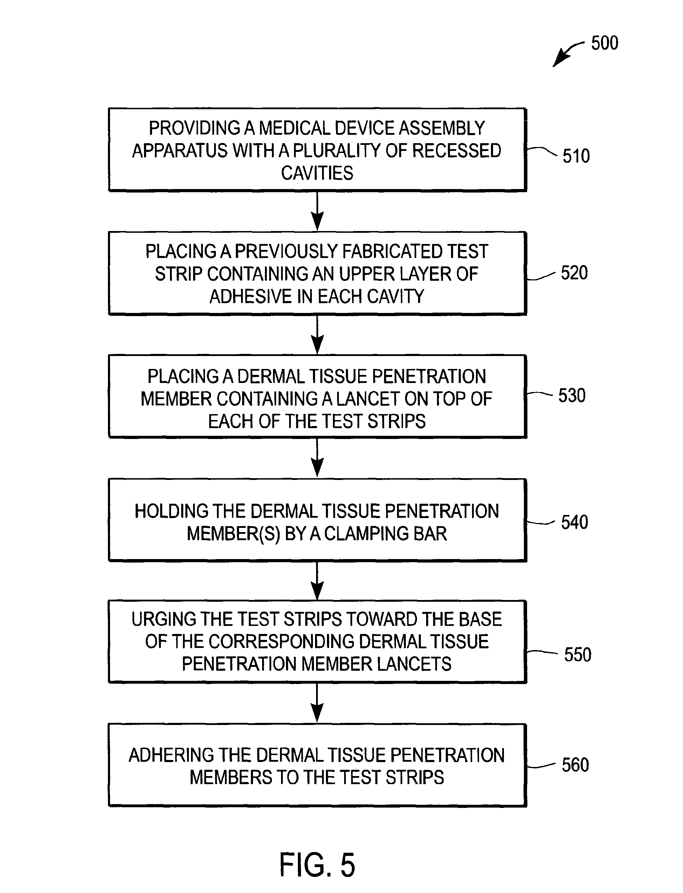

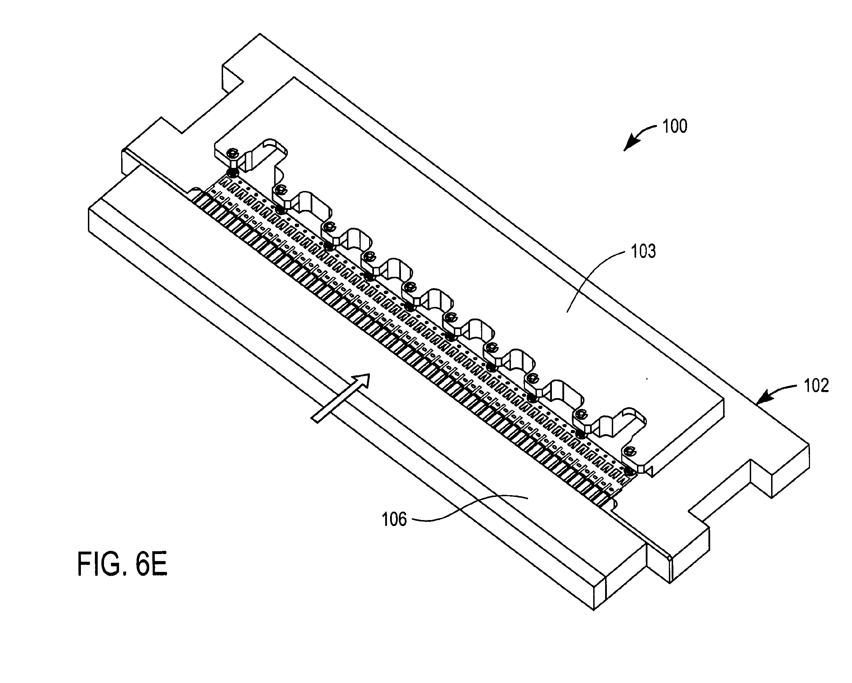

Referring again to Figure 1, body 102 of assembly apparatus 100 includes a first side 108, a second side 110, a first end 112, a second end 114, an upper surface 116 and a lower surface 118. First side 108 includes a plurality of protrusion guides 119 which may be, for example, hollow, tubular-shaped for the plurality of protrusions 107 to move through. The function of protrusions 107 is to move through protrusion guides 119 thereby pushing strips positioned in recess 120 into alignment with dermal tissue penetration members 202 during the manufacturing process, as will be described in more detail below (see Figures 5 and 6E). The cross section of protrusion guides 119 are shaped to accommodate the cross-sectional shape of protrusions 107.

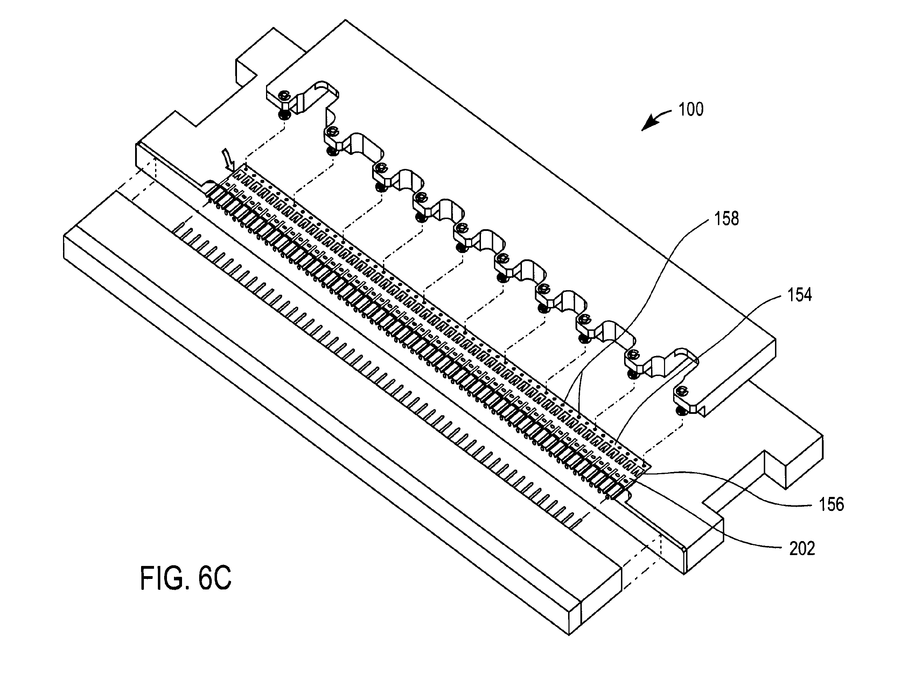

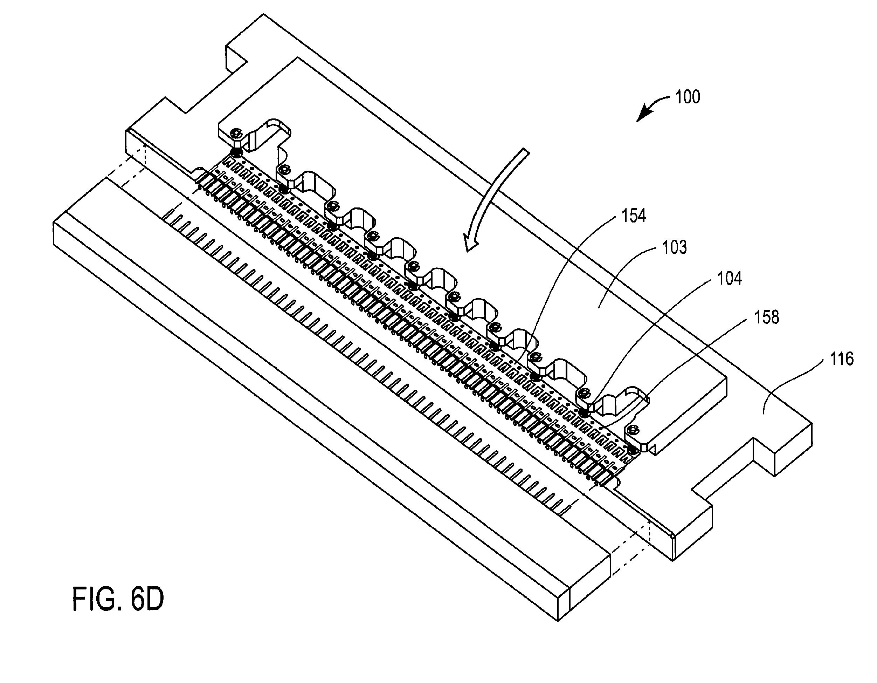

Adjacent to protrusion guides 119 is a plurality of recesses 120 and groove 122 which may be, for example, elongate in shape, on upper surface 116 running from first end 112 to second end 114 (i.e., in the X direction of Figure 1) substantially parallel to first side 108. Adjacent to groove 122 are a plurality of locating pin receiving holes 126. The function of locating pin receiving holes 126 is to align and secure clamping bar 103 through locating pins 104 to body upper surface 116, as will be described in more detail below (see Figures 5, 6C and 6D).

Recesses 120 each contain at least one recess wall 129 approximately perpendicular to groove 122 (i.e., in the Y direction, see Figure 1). Recess 120 is configured (e.g., sized, shaped and/or orientated) to receive and to removably retain a test strip 204 (illustrated in Figures 2A and 2B as part of integrated medical device 200) at least partially therein. The number of recesses 120 can range from 10 to 100 or more and more usually ranges from 20 to 50. The width of recess 120 (i.e., in the X direction) is marginally larger (e.g., about 1-3%) than the width of test strip 204 such that test strip 204 fits snugly therein. This snug fit beneficially minimizes side-to-side movement of the strip during the integrated medical device assembly process (see Figure 5) such that alignment between test strip 204 and dermal tissue penetration member 202 in the X direction is maintained.

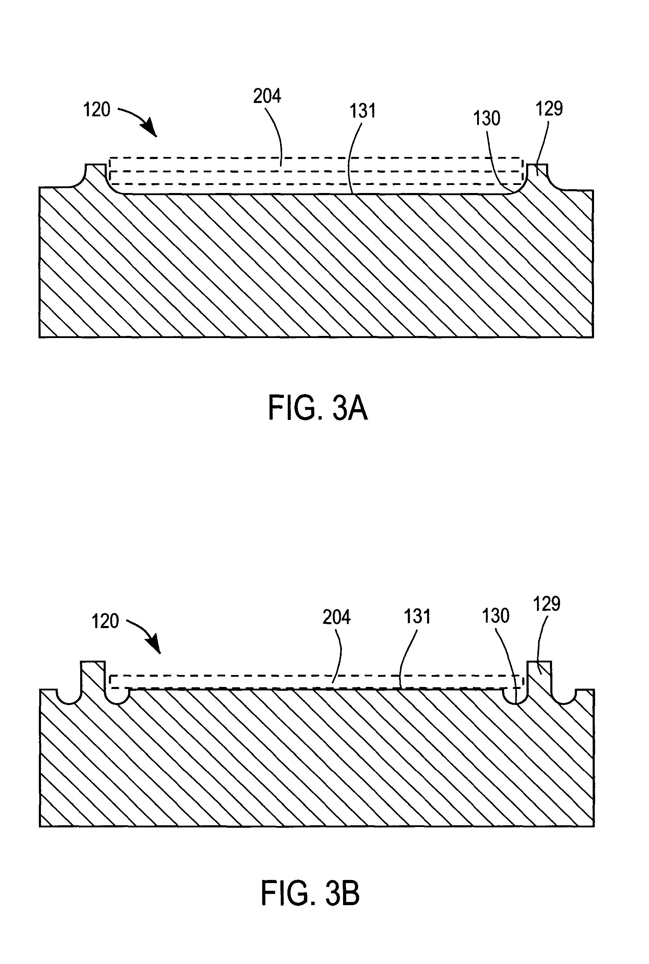

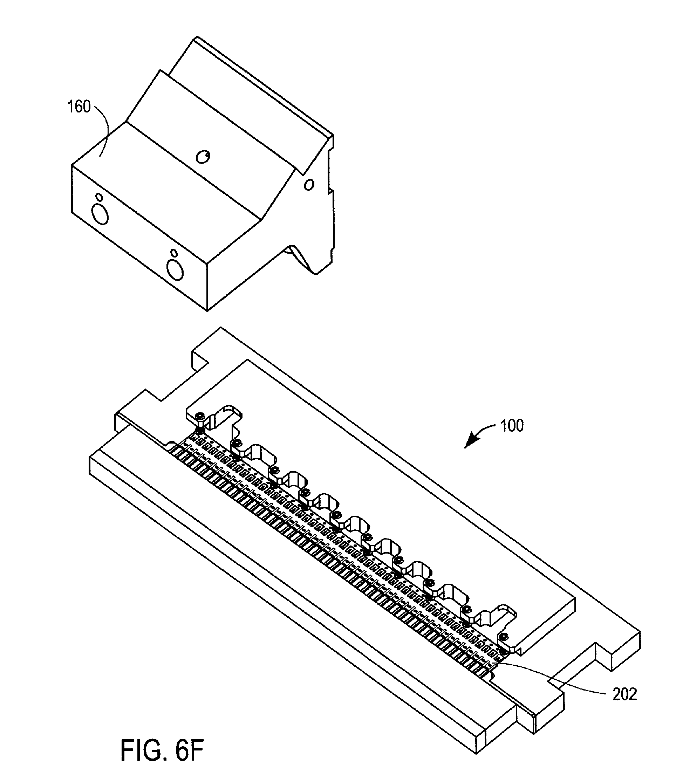

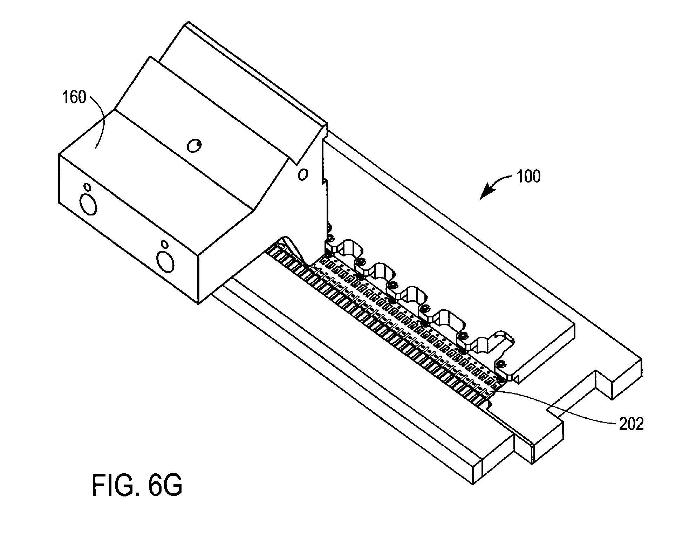

Recesses 120 can be formed by processes known to those skilled in the art including, but not limited to, spark erosion and electrical discharge machining (EDM). Types of EDM include, for example, wire, sinker and small hole EDM. Cross-sectional side views of recess 120 are shown in Figures 3A and 3B. Recess 120 includes at least one corner 130 which, in the embodiment of Figure 3A is a rounded inner corner bounded by recess wall 129 and a recess base surface 131. An exemplary embodiment of recess 120 is shown in cross-section in Figure 3A. In this embodiment, test strip 204 within recess 120 contacts a region on corner 130 but does not contact recess base surface 131. In other words, test strip 204 is held remote from recess base surface 131 by corner 130 which may be, for example, a rounded inner corner. Figure 3B illustrates another exemplary embodiment of recess 120 in which corners 130 are wire eroded to form a depression of approximate semi-circular cross section bounded by recess wall 129 and recess base surface 131 to allow test strip 204 to lie flat within recess 120. This beneficially allows close contact of test strip 204 with recess base surface 131, ensuring complete and even adhesion between test strip 204 and dermal tissue penetration member 202 during the heat seal step in process 500 (see Figures 5, 6F and 6G).

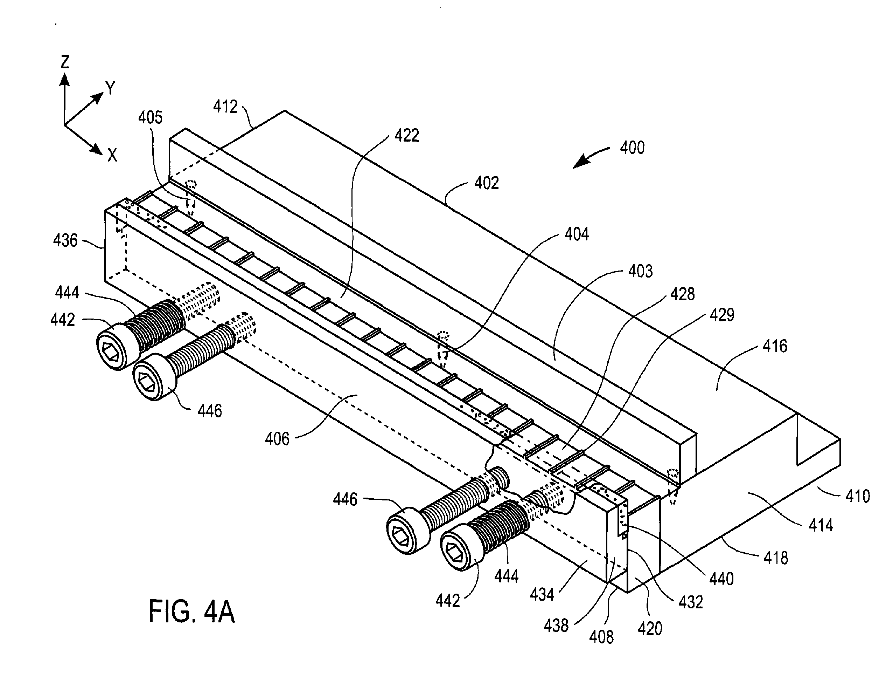

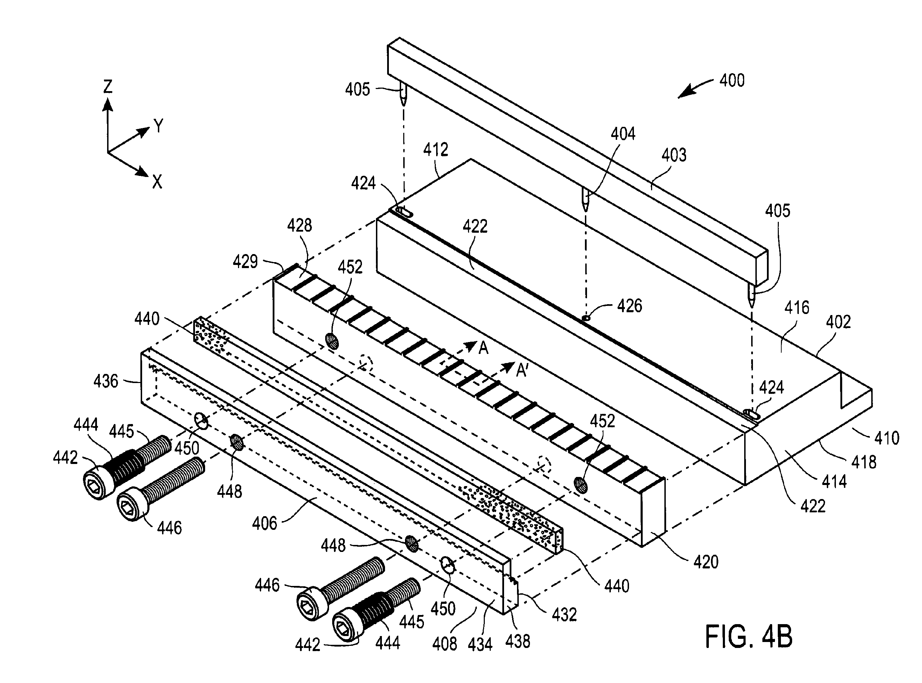

Figures 4A and 4B are perspective and exploded perspective views, respectively, of a medical device assembly apparatus 400 according to another exemplary embodiment of the present invention. Assembly apparatus 400 includes a body 402, a detachable clamping bar 403 with a central locating pin 404, two outer locating pins 405 and a detachable spring-loaded test strip pusher plate 406. Assembly apparatus 400 is generally rectangular in shape and can be formed of metal or any material that can withstand a temperature ranging from about 95°C to 150°C.

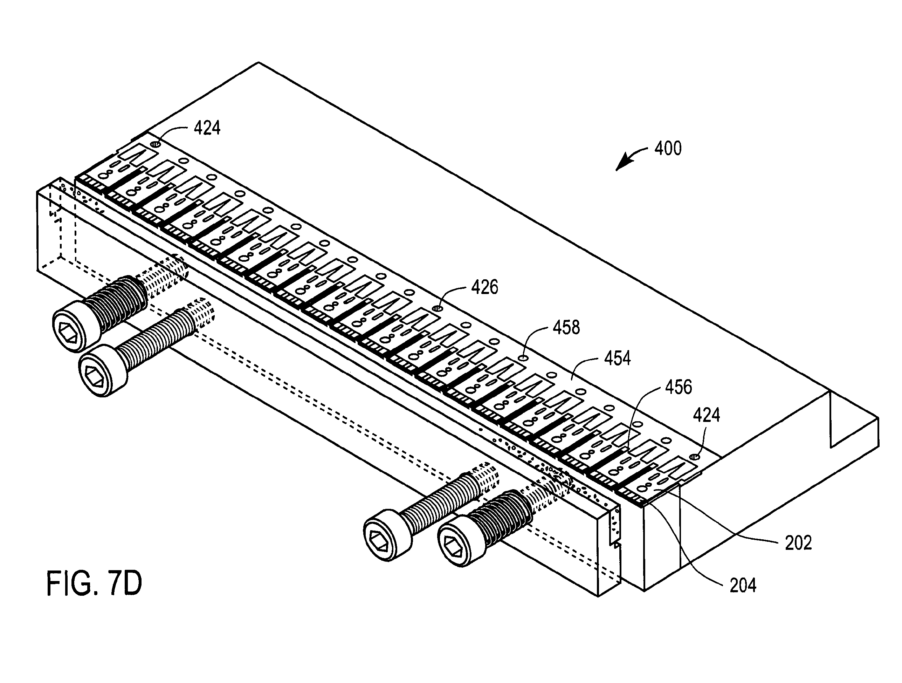

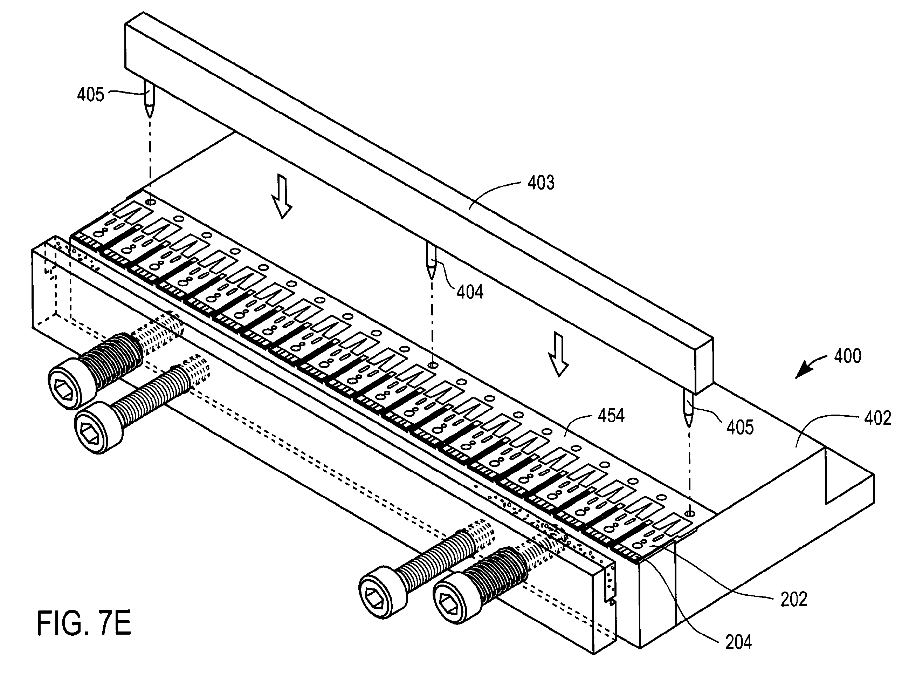

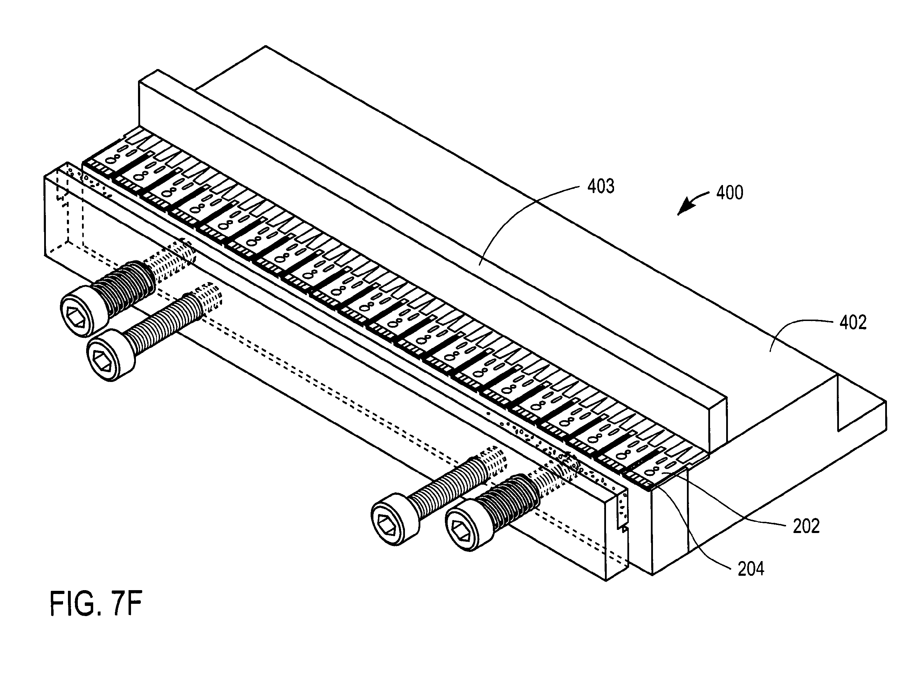

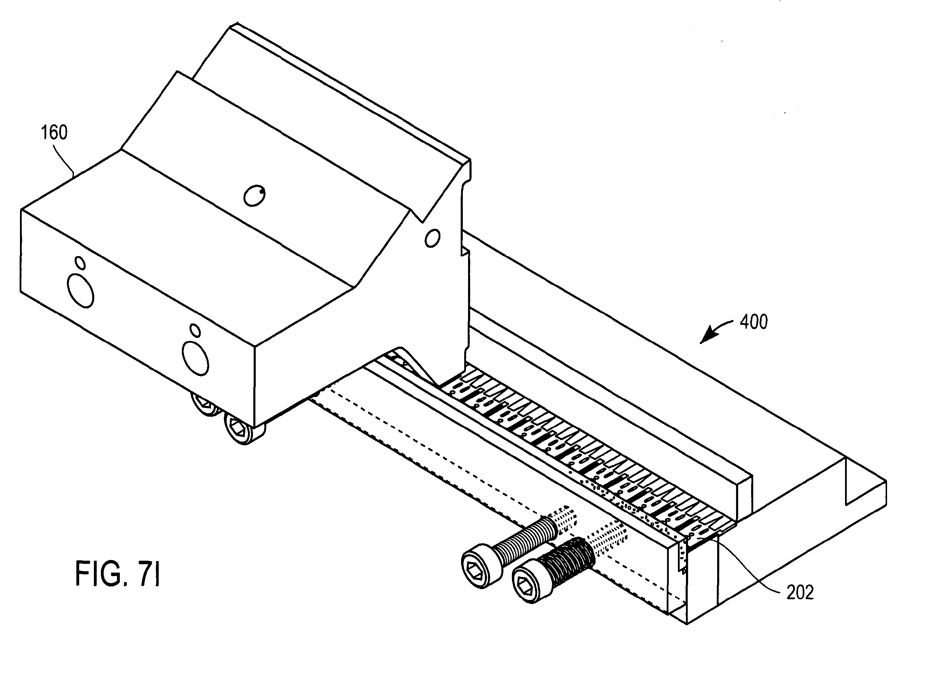

Assembly apparatus body 402 includes a first side 408, a second side 410, a first end 412, a second end 414, an upper surface 416 and a lower surface 418. Second side 410 can include a stepped shape for securing assembly apparatus 400 in a heat-sealing apparatus prior to the integrated medical device assembly process. Adjacent to first side 408 is an elongate recess-containing member 420 and an elongate groove 422 on upper surface 416 running from first end 412 to second end 414 (i.e., in the X direction, see Figures 4A and 4B) substantially parallel to recess-containing member 420. Adjacent to groove 422 are outer locating pin slots 424 near to each of first and second ends 412 and 414. Also adjacent to groove 422 in substantially the center of body 402 is a central locating pin receiving hole 426. The function of outer locating pin slots 424 and central locating pin receiving hole 426 is to align and secure clamping bar 403 through central locating pin 404 and outer locating pins 405 to body upper surface 416, as will be described in more detail below (see Figures 5, 7E and 7F).

Recess-containing member 420 includes a plurality of recesses 428 each containing at least one recess wall 429 approximately perpendicular to groove 422 (i.e., in the Y direction, see Figures 4A and 4B). Recess 428 is configured (e.g., sized, shaped and/or orientated) to receive and to removably retain a test strip 204 (illustrated in Figures 2A and 2B as part of integrated medical device 200) at least partially therein. The number of recesses 428 can range from 10 to 100 and more and usually ranges from 20 to 50. The width of recess 428 (i.e., in the X direction) is marginally larger (e.g., about 1-3%) than the width of test strip 204 such that test strip 204 fits snugly therein. This snug fit beneficially minimizes side-to-side movement of the strip during the integrated medical device assembly process (see Figure 5) such that alignment between test strip 204 and dermal tissue penetration member 202 in the X direction is maintained.

Recess-containing member 420 is securely attached to body 402 by means or processes known to those skilled in the art including, for example, bolting, dowelling and welding. Recess-containing member 420 is fabricated separately from body 402 so that recesses 428 can be formed by processes known to those skilled in the art including, but not limited to, spark erosion and electrical discharge machining (EDM). Types of EDM include, for example, wire, sinker and small hole EDM. The exemplary embodiments of recess 428 shown in Figures 3A and 3B can also be used in assembly apparatus 400.

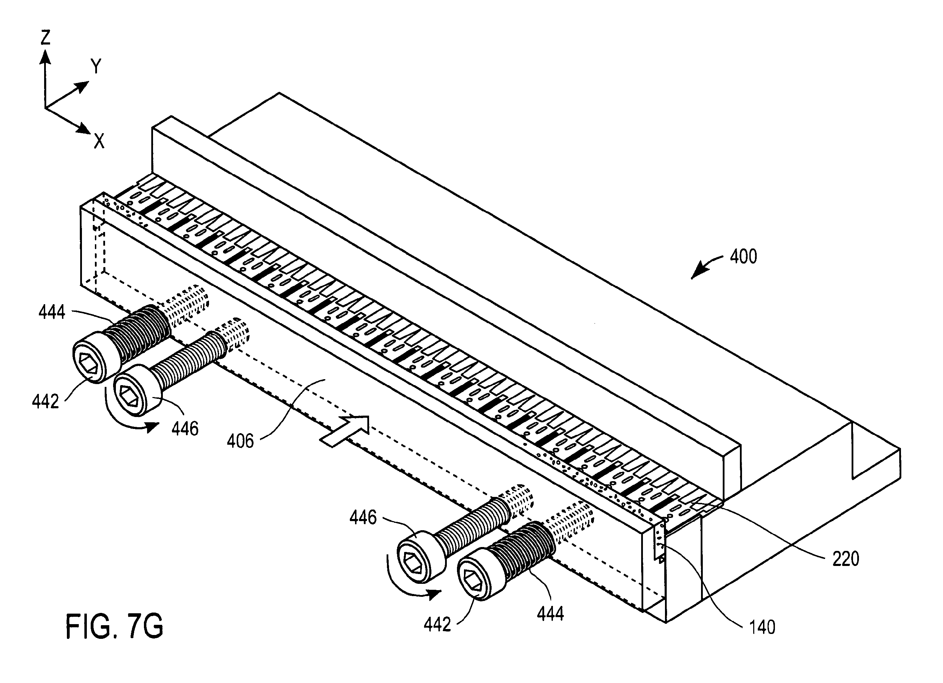

Referring again to Figures 4A and 4B, pusher plate 406 includes a plate proximal side 432 facing recess-containing member 420, a plate distal side 434, a first end 436 and a second end 438. Plate proximal side 432 includes a resiliently deformable band 440 along the entire length of plate 406 from first end 436 to second end 438 and extending to approximately half the height and width of pusher plate 406. Deformable band 440 contacts test strips 204 as pusher plate 406 is urged against recess-containing member 420, as will be described below (see Figures 5 and 7G).

Deformable band 440 can be formed of any resiliently deformable material known to those skilled in the art including, but not limited to, Styrofoam materials, elastomeric materials, silicone materials, latex materials, polymeric materials, polyurethane materials and any combination thereof. Deformable band 440 is detachably adhered to pusher plate 406 with semi-permanent adhesive to allow for removal when deformable band 440 is no longer functional, is soiled or is damaged. Any suitable adhesive known to those skilled in the art can be employed for this purpose including, but not limited to, pressure sensitive adhesives, cold-seal adhesives, heat-seal adhesives and releasable adhesives available from, for example, 3M, Basic Adhesives and Avery Dennison.

Referring to Figure 4B, pusher plate 406 further includes at least one outer screw 442 with a spring 444 in surrounding relation to outer screw threads 445 and at least one inner screw 446. A non-threaded outer screw plate hole 450 allows movement of pusher plate 406 relative to outer screw 442. Outer screw 442 is anchored in recess-containing member 420 through an outer screw threaded body hole 452 that is aligned with non-threaded outer screw plate hole 450. Inner screws 446 can move through the width of pusher plate 406 by threaded inner through screw hole 448. Outer screw(s) 442 and inner screw(s) 446 are positioned inward from first end 436 and second end 438 approximately one quarter and one third of the length, respectively, of pusher plate 406 running in the X direction. Outer screw 442 and inner screw 446 are also positioned in pusher plate 406 below deformable band 440 such that movement of pusher plate 406 with respect to recess-containing member 420 on outer screw 442 and inner screw 446 is not impeded by deformable band 440. Outer screw threaded body holes 452 are also included in recess-containing member 420 for receiving outer screws 442. Outer screws 442 are screwed into recess-containing member 420 through outer screw threaded body holes 452 to a depth sufficient to allow movement of plate pusher 406 away from recess-containing member 420 and to allow compression of springs 444. Inner screws 446 can touch but do not penetrate recess-containing member 420.

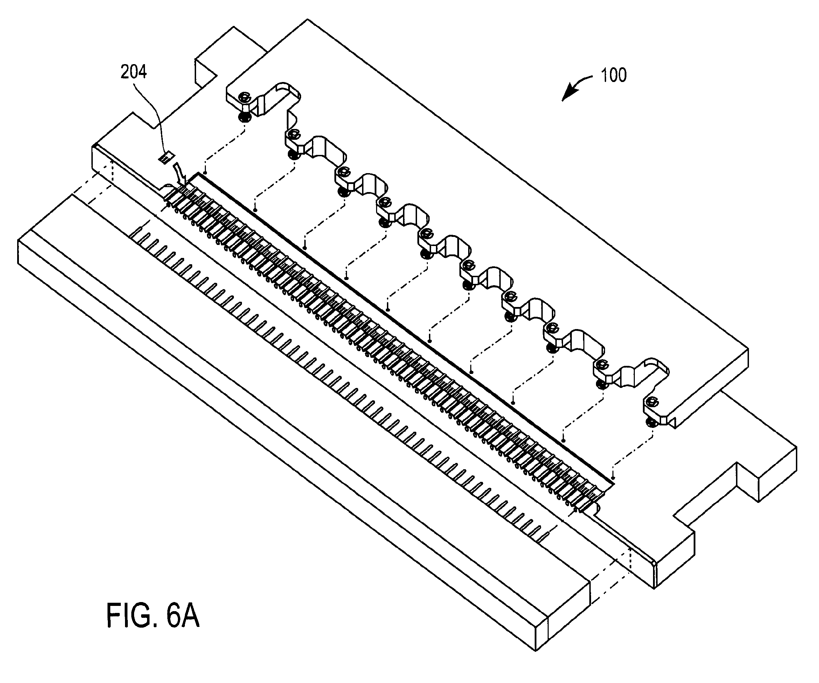

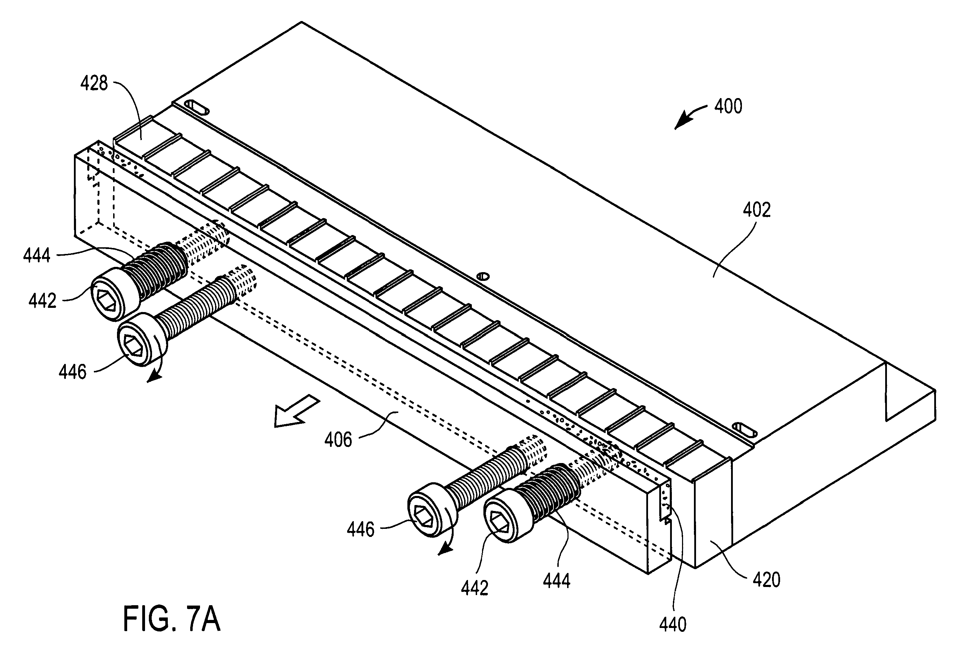

Figure 5 is a flow chart illustrating a sequence of steps in a process 500 for manufacturing a plurality of integrated medical devices according to an exemplary embodiment of the present invention. Process 500 is described below utilizing Figures 6A-6I and 7A-7I (schematic, perspective views depicting various stages of process 500). Process 500 will first be described utilizing assembly apparatus 100 shown in Figures 6A-6I and then will be described utilizing assembly apparatus 400 shown in Figures 7A-7I.

Process 500 includes first providing an assembly apparatus 100, as set forth in step 510 of Figure 5 (see Figure 1). The provided assembly apparatus 100 includes a body 102, a clamping bar 103 with a plurality of locating pins 104, and a pusher plate 106 with a plurality of protrusions 107 which may be, for example spring-loaded. Body 102 further includes a first side with a plurality of protrusion guides 119 for the protrusions 107 to move therethrough. Adjacent to protrusion guides 119 is a plurality of recesses 120 configured to receive and to removably retain test strips 204 at least partially therein.

Next, as set forth in step 520, a previously fabricated test strip 204 with an exposed upper heat seal adhesive layer is placed in each recess 120 in assembly apparatus 100 (see Figure 6A). Test strips 204 used in this process can be manufactured, for example, by web processes as disclosed in U.S. Patent Publication Nos. 2002-0168290 and 2003-0211619 or by screen printing processes as disclosed in International Application No. PCT/GB03/04656 (published as WO 04/040287 on May 13, 2004).

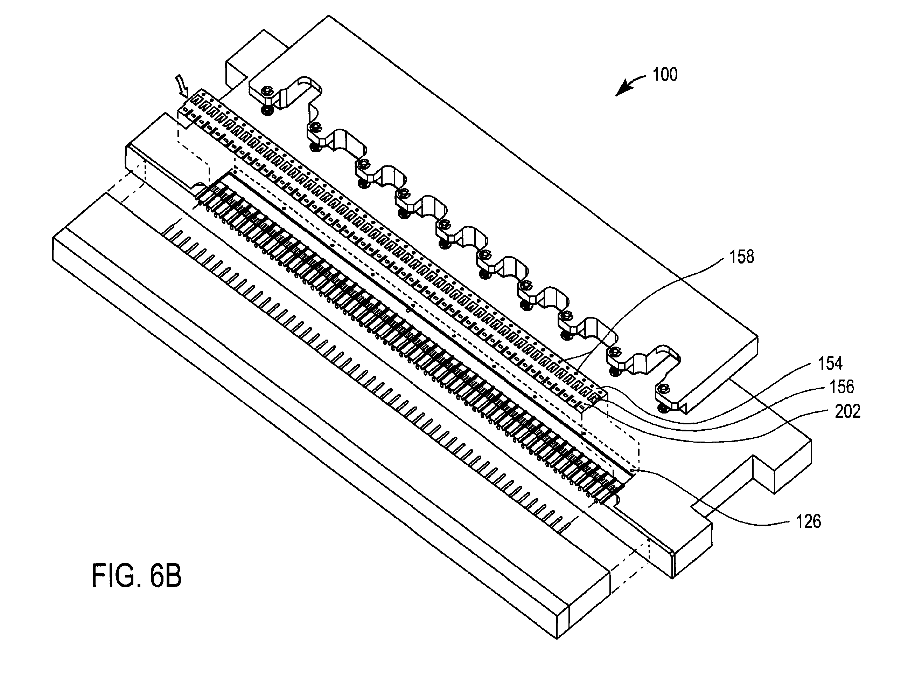

As set forth in step 530, a set of 10 to 50 dermal tissue penetration members 202 attached to a common bandolier 154 through tabs 156 is next placed on top of test strips 204 in assembly apparatus 100 such that at least one bandolier hole 158 is aligned with at least one locating pin receiving hole 126 (see Figures 6B-6C).

Subsequently, clamping bar 103 is attached to body upper surface 116 by placing locating pins 104 through bandolier holes 158 and locating pin receiving holes 126, thereby securing bandolier 154 (see Figures 6D), as set forth in step 540. Locating pins 104 beneficially securely hold bandolier 154 in place to ensure that there is minimal movement of dermal tissue penetration members 202 in the X, Y and Z directions during step 560 (see below).

As set forth in step 550, pusher plate 106 is urged toward body 102, causing test strips 204 to be pushed toward body 102 in the Y direction by protrusions 107 (not shown). Protrusions 107 continue to push test strips 204 until the reaction areas on test strips 204 are aligned with a lancet base 222 (see Figure 6E; strips and lancet base not shown). Movement of protrusions 107 in the Y direction is optionally guided by protrusion guides 119. Protrusions 107 are spring loaded to accommodate variations in test strip length while ensuring that the strips are fully pushed against lancet base 222.

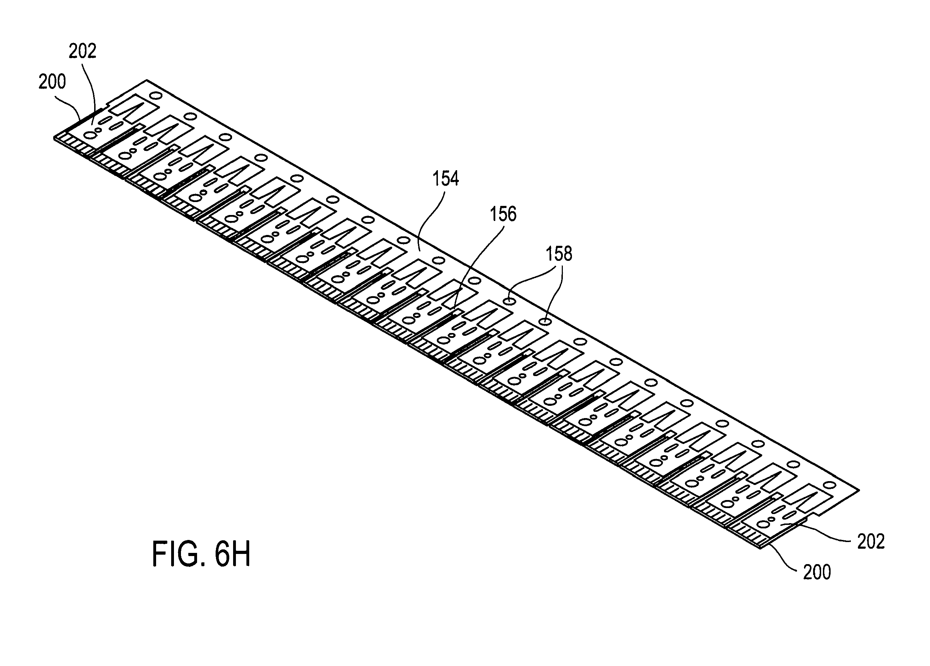

Next, assembly apparatus 100 is placed in a heat sealing apparatus and dermal tissue penetration members 202 are adhered to test strips 204 by a heat sealer 160, as set forth in step 560 (see Figures 6F-6G). Any heat sealer known to those skilled in the art can be used in this step. Heat sealer 160 seals 2 to 20 medical devices at a time and more usually seals 5 to 10 at one time. Typical temperature, pressure and dwell times (i.e., time that the heat sealer contacts the dermal tissue penetration member) for heat sealer 160 range from 95-150°C, 15-40 N per lancet, and 1-5 seconds, respectively. The assembled integrated medical devices 200 attached to bandolier 154 (see Figure 6H) are then removed from assembly apparatus 100 for further processing, i.e. for singulation by cutting through tabs 156 that connect dermal tissue penetration member 202 to the bandolier 154.

When assembly apparatus 400 is used in process 500, process 500 includes first providing an assembly apparatus 400, as set forth in step 510 of Figure 5 (see Figure 7A). The provided assembly apparatus includes a body 402, a clamping bar 403 with a central locating pin 404 and outer locating pins 405 for attaching clamping bar 403 to body 402, and a pusher plate 406 which may be, for example spring-loaded. Body 402 includes a recess-containing member 420 containing a plurality of recesses 428 for receiving test strips therein. The pusher plate 406 includes an optional resiliently deformable band 440 for contacting the test strips during the manufacturing process. Pusher plate 406 further includes at least one outer screw 442 with a spring 444 in surrounding relation to outer screw threads 445 and at least one inner screw 446. Pusher plate 406 can move relative to outer screw 442. Outer screw 442 is anchored in recess-containing member 420 and remains stationary during process 500. Inner screw 446 moves through pusher plate though a threaded inner screw hole 448 and touches but does not penetrate recess-containing member 420. In step 510, pusher plate 406 has been moved away from recess-containing member 420 by turning inner screws 446 clockwise. This allows placement of test strips 204 into recesses 428 (see step 520). Turning inner screws clockwise causes inner screws 446 to push against recess-containing member 420, resulting in movement of pusher plate 406 away from recess-containing member 420 and compression of springs 444. Pusher plate 406 is now spring-loaded in preparation for assembling integrated medical devices.

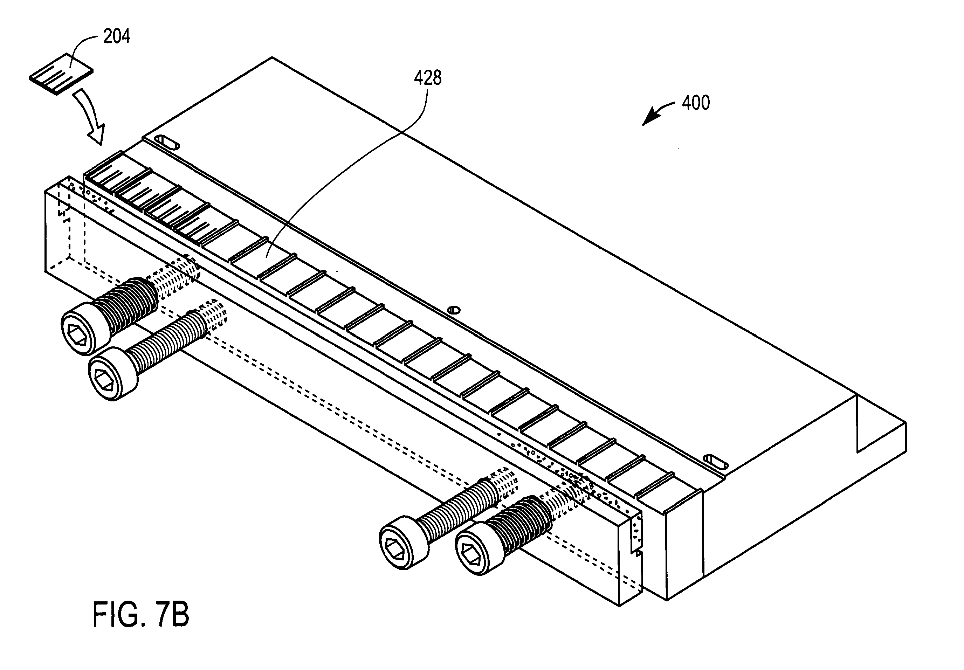

Next, as set forth in step 520, a previously fabricated test strip 204 with an exposed upper heat seal adhesive layer is placed in each recess 428 in assembly apparatus 400 (see Figure 7B).

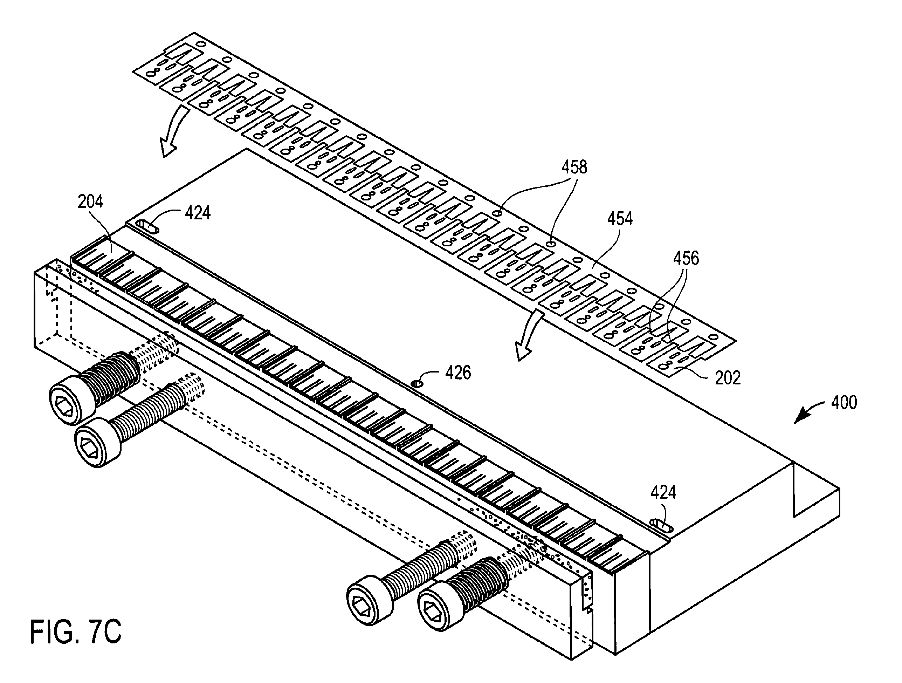

As set forth in step 530, a set of 10 to 50 dermal tissue penetration members 202 attached to common bandolier 454 through tabs 456 is next placed on top of test strips 204 in assembly apparatus 400 such that at least one bandolier hole 458 is aligned with central locating pin receiving hole 426 and at least one outer locating pin slot 424 (see Figures 7C-7D).

Subsequently, clamping bar 403 is attached to body upper surface 416 by placing central locating and outer locating pins 404 and 405 through bandolier holes 458 and outer locating pin slots 424 and central locating pin receiving hole 426, thereby securing bandolier 454 (see Figures 7E-7F), as set forth in step 540. Central locating pin 404 fits securely into body 402 of assembly apparatus 400, whereas at least one outer locating pin 405 fits into outer locating pin slots 424 in body 402, allowing outer locating pins 405 to move as needed during the manufacturing process. Central locating pin 404 beneficially securely holds bandolier 454 in place to ensure that there is minimal movement of dermal tissue penetration members 202 in the X direction during step 560. The combination of a fixed central locating pin 404 and moveable outer locating pins 405 beneficially improves the alignment tolerance for the dermal tissue penetration members relative to the test strips by allowing the penetration members to move on either side of the central locating pin rather than moving the entire length of the bandolier. This configuration therefore effectively halves the alignment tolerance in the X direction.

As set forth in step 550, test strips 204 are pushed toward body 402 in the Y direction by pusher plate 406 such that the reaction area on test strips 204 are aligned with lancet base 222 (see Figure 7G; strips and lancet base not shown). Movement of pusher plate 406 in the Y direction is achieved by turning inner screws 446 counterclockwise to release springs 444. As pusher plate 406 moves in the Y direction, deformable band 440 contacts test strips 204, subsequently pushing strips into position. Deformable band 440 beneficially accommodates variations in test strip length while ensuring that the strips are fully pushed against the base of lancet 220.

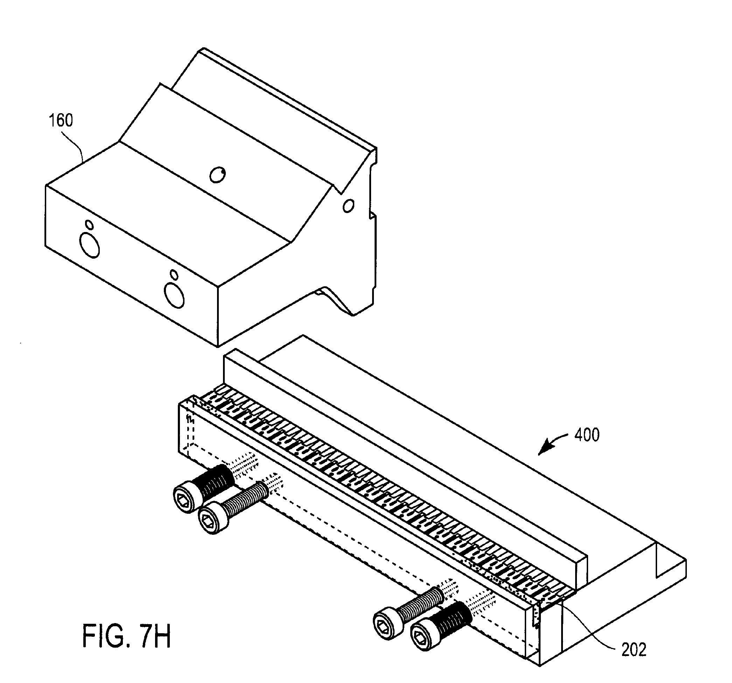

Next, assembly apparatus 400 is placed in a heat sealing apparatus and dermal tissue penetration members 202 are adhered to test strips 204 by a heat sealer 160, as set forth in step 560 (see Figures 7H-7I). Any heat sealer known to those skilled in the art can be used in this step. Heat sealer 160 seals 2 to 20 medical devices at a time and more usually seals 5 to 10 at one time. Typical temperature, pressure and dwell times (i.e., time that the heat sealer contacts the dermal tissue penetration member) for heat sealer 160 range from 95-150°C, 15-40 N per lancet, and 1-5 seconds, respectively. The assembled integrated medical devices 200 attached to bandolier 154 (see Figure 6H) are then removed from assembly apparatus 400 for further processing, i.e. for singulation by cutting through tabs 156 that connect dermal tissue penetration member 202 to the bandolier 154.

Each of the steps of process 500 can be performed, for example, either manually by a user or with the aid of a mechanical and/or electrical device.

Once apprised of the present disclosure, one skilled in the art will recognize that a variety of medical devices can be beneficially manufactured according to the present invention. Such medical devices include, but are not limited to, integrated medical devices that include a combination of a test strip and a lancet, examples of which are described in the aforementioned International Application No. PCT/GB01/05634 (published as WO 02/49507 on June 27, 2002) and U.S. Patent Publication No. 2002-0168290, both of which are fully incorporated herein by reference. One skilled in the art will also recognize that such test strips may have, but are not limited to, an electrochemical or photometric configuration. For illustrative purposes only, medical devices in various figures of the present disclosure were depicted as having an electrochemical configuration.

Moreover, those skilled in the art will appreciate that medical devices according to embodiments of the present invention can be adapted for the measurement of, for example, glucose, ketones, glycated albumin, coagulation parameters and cholesterol of a sample.

In addition, one skilled in the art will also recognize that medical devices according to the present invention may be contained within a combined sample collection and metering system designed for *in-situ* testing. Examples of such systems designed for *in-situ* testing are disclosed in International Patent Application No. PCT/US01/07169 (published as WO 01/64105 A1 on September 7,2001) and International Patent Application No. PCT/GB02/03772 (published as WO 03/015627 A1 on February 27, 2003), each of which is fully incorporated herein by reference.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An integrated medical device assembly apparatus comprising:
a body including:
a proximal end; and
a distal end;
a detachable clamping bar; and
a pusher plate,
wherein the proximal end includes a plurality of recesses for receiving and removably retaining a plurality of test strips.

2. The integrated medical device assembly apparatus of Claim 1, wherein said pusher plate includes a plurality of spring loaded protrusions for contacting said plurality of test strips retained within said recesses.

3. The integrated medical device assembly apparatus of Claim 2, wherein said pusher plate includes a resiliently deformable band adapted to force said plurality of test strips retained into the recesses.

4. The integrated medical device assembly apparatus of Claim 3, wherein said clamping bar includes at least one pin for attaching to said body.

5. An integrated medical device for use in detecting the presence of analytes in blood, said integrated medical device comprising:
a test strip manufactured using a web process;
a first heat activated bonding layer positioned over said first test strip substrate;
a tissue penetration member affixed to said test strip by positioning said test strip and bonding layer in a recessed cavity, placing said tissue penetration member over said bonding layer, clamping said tissue penetration member in place using a clamping bar, forcing said test strip into said recess using a pusher plate and heating said test strip, bonding layer and penetration member to a predetermined temperature.

6. An integrated medical device according to Claim 5 wherein said predetermined temperature is between 95°C and 150°C.

7. An integrated medical device according to Claim 6, wherein said tissue penetration member includes a lancet.

8. An integrated medical device according to Claim 7, wherein a notch in the distal end of said heat activated bonding layer, a first side of said test strip and a first side of said tissue penetration member form a chamber positioned to receive fluid from said lancet.
